# EUROPEAN PATENT SPECIFICATION

(11) **EP 3 385 334 B1**
(45) Date of publication and mention of the grant of the patent: **26.08.2020**
(21) Application number: 17164705.0
(22) Date of filing: 04.04.2017
(51) Int. Cl.: C09B 61/00, C12M 1/00, C12N 1/22

(54) **METHOD FOR EXTRACTING HYDROPHOBIC COMPOUNDS**
VERFAHREN ZUR EXTRAKTION VON HYDROPHOBEN VERBINDUNGEN
PROCÉDÉ D'EXTRACTION DE COMPOSÉS HYDROPHOBES

(43) Date of publication of application: 10.10.2018
(73) Proprietor: SSC Strategic Science Consult GmbH, 22761 Hamburg (DE)
(72) Inventor: Kerner, Martin, 22761 Hamburg (DE)
(74) Representative: Uexküll & Stolberg

(56) References cited:
- CN-A- 105 441 502
- DORINDE M M KLEINEGRIS ET AL: "Continuous production of carotenoids from", ENZYME AND MICROBIAL TECHNOLOGY, STONEHAM, MA, US, vol. 48, no. 3, 12 November 2010 (2010-11-12), pages 253-259, XP028138959, ISSN: 0141-0229, DOI: 10.1016/J.ENZMICTEC.2010.11.005 [retrieved on 2010-11-18]
- Karolina Sliwa ET AL: "Micelle-mediated extraction of elderberry blossom by whey protein and naturally derived surfactants INTRODUCTION", , 1 January 2013 (2013-01-01), XP055383151, Retrieved from the Internet: URL:http://www.actabp.pl/pdf/4_2013/803.pd f [retrieved on 2017-06-20]
- KAROLINA ?LIWA ET AL: "A micelle mediated extraction as a new method of obtaining the infusion of Bidens tripartita", ACTA BIOCHIMICA POLONICA, vol. 63, no. 3, 1 January 2016 (2016-01-01), pages 543-548, XP055383156, PL ISSN: 0001-527X, DOI: 10.18388/abp.2015_1223
- CHOI M P K ET AL: "Pressurized liquid extraction of active ingredients (ginsenosides) from medicinal plants using non-ionic surfactant solutions", JOURNAL OF CHROMATOGRAP, ELSEVIER, AMSTERDAM, NL, vol. 983, no. 1-2, 3 January 2003 (2003-01-03), pages 153-162, XP004398957, ISSN: 0021-9673, DOI: 10.1016/S0021-9673(02)01649-7
- ARPAN PARMAR ET AL: "Interaction and solubilization of some phenolic antioxidants in Pluronicmicelles", COLLOIDS AND SURFACES. B, BIOINTERFACES, ELSEVIER, AMSTERDAM, NL, vol. 86, no. 2, 7 April 2011 (2011-04-07), pages 319-326, XP028223129, ISSN: 0927-7765, DOI: 10.1016/J.COLSURFB.2011.04.015 [retrieved on 2011-04-16]
- MATTHEW K. FLESHMAN ET AL: "Carotene and Novel Apocarotenoid Concentrations in Orange-Fleshed Cucumis melo Melons: Determinations of [beta]-Carotene Bioaccessibility and Bioavailability", JOURNAL OF AGRICULTURAL AND FOOD CHEMISTRY, vol. 59, no. 9, 11 May 2011 (2011-05-11), pages 4448-4454, XP055383435, ISSN: 0021-8561, DOI: 10.1021/jf200416a

## Description

The invention relates to a new method for the micelle-mediated in situ extraction of hydrophobic compounds from cells. The method comprises culturing cells in an aqueous medium such that hydrophobic substances are produced within the cells; adding to said medium a surfactant to form micelles containing the hydrophobic substances; contacting said medium in a counter current extraction device with a flow of a liquid surfactant; increasing the temperature in the extraction device to a temperature which is higher than the surfactant's cloud point temperature to separate an aqueous phase containing the cells from a surfactant-rich phase containing the micelles; and removing the surfactant-rich phase from the medium and circulating it back into the extraction device.

### BACKGROUND OF THE INVENTION

The interest in microalgae products is constantly growing. Different species of microalgae are cultivated in order to produce valuable compounds like b-carotene (Dunaliella salina), astaxanthine (Haematococcus pluvialis) and the like. Special attention is given to the extraction of these valuable components from algae cultures. State of the art processing of microalgae currently includes energy consuming cell harvesting, dewatering, cell disruption and lipid extraction from the algae biomass using classical extraction methods. Commonly applied extraction methods use supercritical fluids like carbon dioxide or organic solvents (mainly n-alkanes like hexane), as well as ultrasonic-assisted extraction procedures. All these techniques have in common that the algae cells are disrupted, followed by a time and cost consuming re-growing step of the algae biomass. None of the described methods allows a simultaneous cultivation of algae cells and an extraction of the hydrophobic target substances (in situ extraction approach). The in situ extraction is generally based on two-phase systems, where one phase is the culture medium containing microorganisms and the other phase is an extractive agent (it can be an organic solvent or a surfactant-containing solution), which is biocompatible with the cultivated organisms. With such a system the extracted substances can be obtained directly from the culture medium or from the cells after permeabilization of their cell walls or cell lysis. In extraction processes, all of these mechanisms are of interest. The dodecane-based extractions from the microalgae D. salina in two-phase bioreactors have been extensively studied by Wijffels and co-workers in a series of publications. They investigated selective extraction of beta-carotene directly from the microalgae culture (Hejazi et al. (2004), Biotechnol. Bioeng. 88, 593-600; Kleinegris et al. (2010), Mar. Biotechnol. 12 14-23). Thereby n-dodecane was found to be efficient for extraction but showed cell toxicity during longer contact times (Kleinegris et al. (2011), Enzyme Microb. Technol. 48, 253-259).

Alternative extraction media for the in situ extraction of algae can be nonionic surfactant water mixtures. The amphiphilic molecules of nonionic surfactants form micellar aggregates in aqueous solutions, when their concentration reaches the surfactants' critical micelle forming concentration (CMC). The hydrophobic head of the molecule is directed to the bulk water and the hydrophobic tails form the hydrophobic micelle core. These solutions form temperature induced two-phase systems. After a temperature increase above the cloud point temperature (CPT), the homogeneous micellar system divides into two phases: a surfactant-rich phase and an aqueous phase. After phase separation of the cloud point system, hydrophobic solutes (solubilized in the micelles) accumulate in the surfactant-rich phase. Some nonionic surfactants show relatively low cloud point temperatures which allows the application of the corresponding cloud point system as two phase extraction media for whole cell bioprocesses. Such a process is called cloud point extraction (CPE). It was demonstrated that the mold Monascus purpureus can be cultivated in Triton X-100/water mixture without significant cell growth inhibition (Wang (2007), Appl. Microbiol. Biotechnol. 75, 1-10). Furthermore, the extraction of the valuable pigments was implemented with permeabilization of the cell membrane. After carrying out the CPE, microbial cells predominantly accumulate in the aqueous phase. Hydrophobic molecules like oleic acid, polycyclic hydrocarbons and other fatty acids were rapidly solubilized in micelles formed by nonionic surfactants.

However, an important drawback of the current methods is the high amount of surfactant that has to be used for cloud point extraction. This high amount of surfactant is required for efficient extraction, but on the other hand it is toxic for the cells and also expensive when industrial-scale cultures are extracted. Accordingly, there is a need for improved methods which are amenable to high culture volumes, reduce the amounts of surfactants and contact time during the extraction process. In addition, there is need for a continuous in situ extraction during cultivation because many substances produced by the cells are metabolized in short time and thus do not accumulate in the culture medium.

These objectives are achieved by the method defined in the enclosed claims. Accordingly, in a first aspect the invention provides a method for the micelle-mediated in situ extraction of hydrophobic compounds from cells, comprising
(a) culturing cells in an aqueous medium such that hydrophobic compounds are produced;
(b) adding to the cell-containing medium a surfactant in a concentration of between 1-200-fold of the surfactant's critical micelle forming concentration (cmc) to form micelles that contain hydrophobic compounds;
(c) conducting the cell-containing medium into a counter current extraction device to provide a medium flow;
(d) contacting the medium flow in said extraction device with a flow of a liquid surfactant which comprises a concentration of more than 200-fold of the surfactant's cmc and which is conducted into said extraction device in counter current direction relative to the medium flow;
(e) increasing the temperature in the said extraction device to a temperature which is higher than the surfactant's cloud point temperature (cpt) to separate an aqueous phase containing the cells from a surfactant-rich phase containing the micelles;
(f) circulating the surfactant-rich phase which has passed through said extraction device back into the extraction device;
(g) and, optionally, continuing to culture the cells in the extracted medium.

The method of the invention is intended to provide for the extraction of hydrophobic compounds from cell cultures during cell culturing (in situ). The extraction does not require cell harvest and a subsequent disruption of the cells. Instead, the cell culture is continuously subjected to extraction by a circulating flow of a highly concentrated surfactant. The circulating surfactant flow is progressively enriched for the hydrophobic compounds by continuously contacting it with the culture volume. The method is based on the inclusion of hydrophobic compounds produced by the cells in micelles, i.e. aggregates of surfactant molecules which are dispersed in a liquid colloid. In aqueous solution micelles are oriented such that the hydrophilic head regions of the surfactant molecules are in contact with the surrounding water, while the hydrophobic tail regions are facing the inside of the micelle. During the process of the invention, the hydrophobic compounds in the medium are included into micelles which shield them from the surrounding water. Another advantage of the method is that the process parameters can be adapted such that after passing through the extraction device, the surfactant concentration in the cell-containing medium is between 1 to 200-fold of the surfactant's cmc which means that the cells can be used for further culturing and can be subjected, at a later stage, to another round of extraction. By using repeating circles of extraction, the yield of the target compounds dramatically increases.

The method of the invention can be used for any type of hydrophobic or lipophilic compound which is produced and optionally secreted from the cells used for culturing. For the purpose of the present invention, the terms "hydrophobic" and "lipophilic" are used interchangeably. Both terms refer to a situation where a compound has a higher solubility in non-polar solvents such as alcohol than in aqueous solutions.

The hydrophobic compound may be a natural or synthetic compound. It may belong to different classes of hydrophobic compounds, such as hydrocarbons, waxes, sterols, ketones, pigments, proteins, or enzymes. It may be a pharmaceutically active compound, such as an antibiotic or antiviral compound, or an anti-inflammatory compound. As representative examples of hydrophobic antibiotics which may be extracted using the method of the invention include macrolides, such as erythromycin, roxythromycin, clarithromycin, etc.; 9-N-alkyl derivatives of erythromycin; midecamycin acetate; azithromycin; flurithromycin; rifabutin; rokitamycin; rifapentine; benzypiperazinyl rifamycins; benzoxazinorifamycin; difficidin; dirithromycin; M-119-a (Kirin Brewery); 3-0-alpha-L-cladinosyldeepoxy rosaramicin; tylosins and acyl demycinosyl tylosins.

It may also be a hydrophobic compound of nutritional value, such as a vitamin or fatty acid. Suitable vitamins include vitamin A, vitamin D, vitamin E and vitamin K and their salts or derivatives. Vitamin D may be selected from vitamin D2, vitamin D3 and their salts or derivatives. Vitamin E may be selected from α, β, γ, or δ-tocopherols, α, β, γ, or δ-tocotrienol and their salts or derivatives, such as tocopheryl sorbate, tocopheryl acetate, or tocopheryl succinate. Vitamin A may be selected from the group consisting of retinol, retinal, retinoic acid, or their salts or derivatives, such as vitamin A acetate, and vitamin A palmitate). Vitamin K may be selected from the vitamin K1, vitamin K2, vitamin K3, vitamin K4, vitamin K5, vitamin K6, vitamin K7, and their salts or derivatives. Suitable fatty acids may be saturated, polyunsaturated, or monounsaturated fatty acids. They may be natural or synthetic in nature. Fatty acids that can be extracted from the cells according to the method of the invention include, but are not limited to, decanoic acid, undecanoic acid, oleic acid, myristic acid, palmitic acid, stearic acid, myrisoleic acid, palmitoleic acid, alpha-linoleic acid, omega-3 fatty acids, such as docosahexaenoic acid, eicosapentaenoic acid, α-linolenic acid, eicosatetraenoic acid, stearidonic acid eicosatrienoic acid, docosapentaenoic acid and glycerol ester derivatives thereof, omega-6 fatty acids, such as linoleic acid, gamma-linolenic acid, eicosadienoic acid, dihomo-gamma-linolenic acid, arachidonic acid, docosadienoic acid, adrenic acid, docosapentaenoic acid and calendic acid), omega-9 fatty acids (e.g., oleic acid, eicosenoic acid, mead acid, erucic acid, precursors of fatty acids, and derivatives of fatty acids.

In one embodiment, the hydrophobic compound is an antioxidant. As used herein, an antioxidant refers to an agent which prevents or delays the oxidation of other compound. Examples of antioxidants include phospholipids, such as soy or egg lecithin, phosphatidyl choline, phosphatidyl ethanolamine, phosphatidyl serine, polyphenols, green tea extracts, grape seed extracts, resveratrol, cinamic acid and salts thereof, ferulic acid and salts thereof, rosemarinic acid and salts thereof, carotenoids (e.g., α-, β-, and γ-carotene, lutein, astaxanthin, and zeaxanthin), curcuminoids such as curcumin, chlorophyllin and salts thereof.

The hydrophobic compound may also be a protein, polypeptide or peptide, which is characterized by a high number of hydrophobic amino acid side chains. Hydrophobic proteins include, for example, bacterial membrane proteins. Suitable therapeutically active hydrophobic proteins include, e.g. interferons, such as betaseron, and fragments thereof.

The method of the invention is broadly applicable to any cells which are known in the art as host organisms for the production of hydrophobic target compounds. The cells can be of eukaryotic or prokaryotic origin. For example, the cells used for culturing can be mammalian cells, e.g. a primary cell or cell line that is derived from a mouse, rat, hamster, dog, or non-human primate. Mammalian cells that are useful for the method of the invention, in particular for the heterologous expression of proteins, include both primary cells (e.g. murine bone marrow cells, haematopoietic stem cells, murine lymphocytes, muscle cells, hepatocytes, and the like) and established cell lines (e.g. Chinese hamster ovary (CHO) cells, monkey kidney cells (COS), baby hamster kidney (BHK) cells, African green monkey kidney (Vero) cells, Madin Darby canine kidney (MDCK) cells, and the like).

In an alternative embodiment, the cells used for the production of hydrophobic target compounds may be fungal cells, and in particular yeast cells. For example, yeast cells which have been derived from the genus Saccharomyces, Schizosaccharomyes, Yarrowia, Kluyveromyces and Pichia have been shown to be useful for the production of hydrophobic target compounds, and in particular for the heterologous expression of hydrophobic proteins. Suitable yeast species for use in the method of the invention are Saccharomyces cerevisiae, Schizosaccharomyes pombe, Yarrowia lipolytica, Kluyveromyces lactis and Pichia pastoris.

In a still further embodiment, the cells used in the method of the invention are insect cells, such as cells of the insect species Aedes aegypti, Autographa californica, Bombyx mori, Spodoptera frugiperda, or Trichoplusia ni.

In view of the ease of handling and the rapid cell growth, the use of bacterial cells or algae cells is particularly preferred herein. Bacterial cells from numerous species have been used in the art for the production of hydrophobic target compounds. For example, strains of the genus Escherichia, Corynebacterium and Bacillus are often used for such purposes. Commonly used species include Escherichia coli, Corynebacterium glutamicum and Bacillus subtilis. In a preferred embodiment, the bacterial cells are E. coli cells, such as cells that have been derived from E. coli strain BL21. In another preferred embodiment, the bacterial cells are phototrophic bacteria, such as cyanobacteria or Rhodospirillales. Cyanobacteria to be used in the process of the invention include, but are not limited to the genera Chroococcus, Gloeocapsa, Gloeobacter, Anabaena, Aphanizomenon, Nostoc, Nodularia, Lyngbya, Oscillatoria, Phormidium, Spirulina, Chroococcidiopsis, Prochloron, Prochlorococcus, Prochlorothrix, and Stigonema. Rhodospirillales that can be employed include, but are not limited to the genera, Phaeospirillum, Rhodospira, Rhodovibrio, Telmatospirillum, Rhodospira und Skermanella.

In another preferred embodiment, the cells that produce the hydrophobic target compounds are algae cells, e.g. microalgae cells. For example, algae cells from the genus Dunaliella, Haematococcus, Chlorella, Coccomyxa, Coelastrella or Scenedesmus may be used. Exemplary algae species that can be used according to the invention include Dunaliella salina, Haematococcus pluvialis, Chlorella vulgaris, Coccomyxa acidophila, Coelastrella striolata and Scenedesmus almeriensis.

In a first step, the cells are cultured in an aqueous medium such that hydrophobic substances are produced and optionally secreted into the medium. The specific parameters of the cell culturing will depend on the cells that have been selected for culturing. Numerous culturing methods have been described for different organisms. In particular, the culturing of bacteria, algae, mammalian cells and yeasts has been described in the art in much detail. Normally, non-phototrophic cells are cultured in a suitable culture medium that comprises all nutrients which are required by the organism. Non-phototrophic cells are usually cultivated in a culturing vessel, such as an aerated stainless steel biofermentor, while phototrophic cells, such as algae or cyanobacteria, are normally cultured in photobioreactors comprising at least a light-transmissive part (e.g. made of glass or translucent plastic) that allows the cells to use sunlight for performing photosynthesis. The cells are grown until a certain cell density is reached. The desired products will in many cases be produced and secreted during the logarithmic growth phase. Where the hydrophobic product is a recombinantly produced protein, it is normally required to provide the cell selected for the process with a copy of the gene encoding the protein on an expression vector. A high number of expression vectors have been described in the art, in particular for the recombinant expression of proteins in bacterial cells. Such vectors may be inducible which means that it is possible to induce the production of the protein by inducing expression of the protein by activating an inducible promoter, e.g. by the addition of an inducer such as IPTG.

The cells are cultured under conditions that allow the hydrophobic substances to be produced and optionally secreted into the medium. Where the cells normally secret the product of choice during their growth, no additional steps are required for accumulating the product in the medium. Where the product is normally stored internally in the cells, it might be preferred to permeabilize the cell wall, e.g. by using a substance like Triton X-100 or saponin or physical methods like ultrasonic sound or magnetic fields.

In a subsequent step, a surfactant is added to the medium that contains the cells. The surfactant is added in a concentration which is 1-200-fold of the surfactant's critical micelle forming concentration (cmc), so that the surfactant forms micelles which encompass the hydrophobic substances, including the target substance. As the concentration of the surfactant in the medium after addition of the surfactant corresponds to the cmc or exceeds the cmc, the surfactant molecules will start to form micelles in the aqueous medium wherein the hydrophobic substances present in the medium will become entrapped in the micelles. According to the invention, the use of non-ionic surfactants is particularly preferred, more preferably one that is not toxic for the cells. It has been found that not only hydrophobic compounds which are normally secreted by the cells are entrapped in the micelles, but also compounds that are normally stored inside the cell. Without wishing to be bound by theory, it is assumed that the surfactant molecules in the medium permeabilize the cell walls to some extent such that part of the hydrophobic compounds normally stored inside the cells are extracted to the medium without affecting the viability of the cell. Alternatively or in addition, it is possible that by entrapping hydrophobic compounds into micelles, the solution equilibrium for these compounds is changed such that part of the part of the hydrophobic compounds normally stored inside the cells is expelled to the surrounding medium.

The specific concentration to be used in this step depends on the detergent selected for the process. The cmc values for detergents are known in the art and are usually provided in the package insert of the respective detergent compound. For example, the cmc of Tween 20 is 0.05 mM. Therefore, if Tween 20 is used in the method of the invention, then between 0.05 and 10.00 mM Tween 20 is added to the culture medium in step (b) of the method of the invention. Under these conditions, sufficient Tween 20 is present in the medium to spontaneously form micelles. In the micelles, the hydrophobic head of the surfactant molecule is directed to the aqueous surrounding of the culture medium, whereas the hydrophobic tails are directed to the inside of the micelle and form its hydrophobic core. Care is taken in step (b) that the surfactant's concentration is high enough to exceed the cmc, but not so high as to lyse or otherwise damage the cells in the medium that produce the hydrophobic target compounds. The toxicity threshold levels can be determined for each detergent-cell combination by routine experimentation.

On the one hand, the selection of the surfactant will depend on the cmc which should allow the cells to grow and replicate in the presence of the surfactant at 1-200-fold of the surfactant's cmc. On the other hand, a surfactant for use in the method of the invention will have a cloud point temperature (cpt) that is not so high as to damage or disrupt the cells that are heated to such temperature in the subsequent step that is performed in the extraction device. Normally, the cpt will be chosen such that it is about 4-20°C, about 4-15°C, about 4-10°C, about 4-8°C, or about 4-5°C higher than the maximal cell culturing temperature of the respective cells to be cultured. Thus, when mesophilic organisms that are cultivated at about 30-35°C are used in the extraction process of the invention, the cpt of the surfactant will be in the range of about 35-42°C, preferably about 39 or 40°C. Surfactants with a higher cpt can be used when extracting compounds from thermophilic microorganisms, such as thermophilic bacteria.

For example, when bacteria or algae are cultured at temperatures of about 32-34°C, suitable surfactants which can be used for the extraction include Silwet L7230 (cpt: 37.4°C), Silwet L7002 (cpt: 39.5°C), Synperonic 91/5 (cpt: 37.9°C), Rokanol NL 5 (cpt: 39°C). When the method of the invention is performed with algae cells, the use of Rokanol NL 5 as a surfactant is preferred. The use of surfactants has the particular advantage that, dependent on the ultimate use of the hydrophobic compounds to be extracted from cells, it is possible to use the extract without separating off the surfactant. In this way, laborious additional purification steps can be omitted. Where the extract resulting from the process is intended for direct use without further separation of the surfactant from the extracted hydrophobic compounds, it may be advantageous to use a surfactant which has regulatory approval for being used as additive in food items, or in cosmetic or pharmaceutical products.

After addition of the surfactant to the medium, the medium is optionally incubated for 1-100 h, preferably 12-24 h, and more preferably 2-6 h, e.g. for about 8 hours, to allow the formation of the micelles and the accumulation of the hydrophobic materials in the micelles. The medium will be incubated at a temperature which is well below the cpt, and preferably about 4-20°C, about 4-15°C, about 4-10°C, about 4-8°C, or about 4-5°C below the cpt of the surfactant.

In the next step, the medium containing the cells and the micelles is conducted into a counter current extraction device, preferably a counter current extraction column. In this way, a liquid medium flow is provided which is conducted through an inlet into the device, such as a column. A typical counter current extraction device, such as a counter current extraction column, for use in the method of the invention has a volume which is at least 200 fold smaller, preferably at least 100 fold smaller, more preferably at least 50 fold smaller, at least 25 fold smaller, at least 20 fold smaller, at least 15 fold smaller, at least 10 fold smaller, at least 5 fold smaller, at least 3 fold smaller, or at least 2 fold smaller than the volume of the culture medium. The smaller the volume of the device in relation to the volume of the culture medium, the lower the flow rate through the counter current extraction device at a given retention time is. For example, if a counter current extraction column has a volume of about 2 L, and a retention time of 2 hours is selected, it will be possible to extract about 1 L medium per hour. When a counter current extraction column having a volume of about 50 L is used with the same retention time, the volume of the medium that can be extracted will be about 25 L per hour. In some preferred embodiments of the invention the counter current extraction device, such as a counter current extraction column, for use in the method of the invention has a volume of between 2-50 L, preferably between 10-40 L, such as 35 L. In other preferred embodiments, the counter current extraction device, such as a counter current extraction column, for use in the method of the invention has dimensions that allow the conduction of about 100 L or more, 200 L or more, or 300 L or more of cell-containing medium through the device per hour. In a preferred embodiment, the counter current extraction device used in the method of the invention is a column has a diameter of between about 0.1-1.0 m, preferably about 0.5 m.

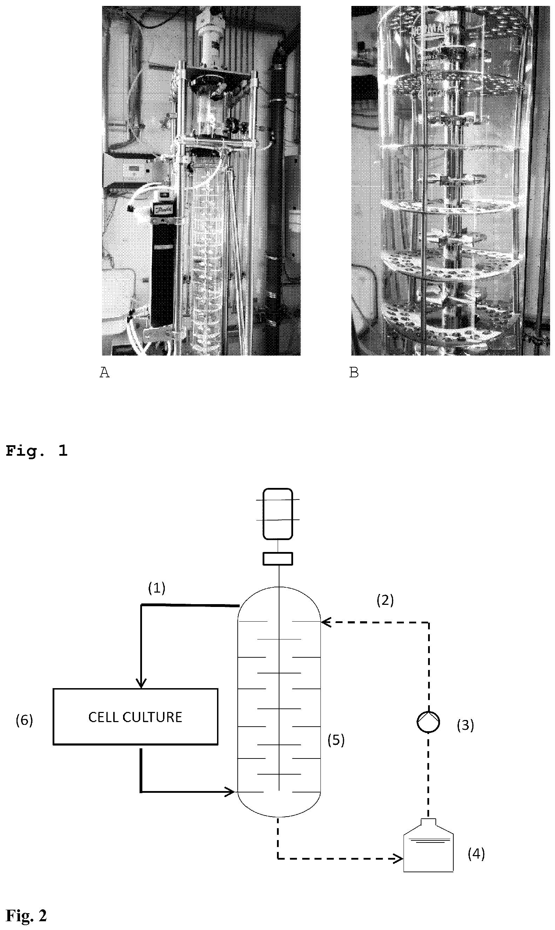

A counter current extraction column for use in the method of the invention is shown in figure 1. A typical extraction column comprises an elongated cylindrical chamber for the extraction, a first inlet for conducting a first liquid into the upper part of the extraction chamber, a second inlet for conducting a second liquid into the lower part of the extraction chamber, a first outlet for discharging the first liquid from the lower part of the extraction chamber, and a second outlet for discharging the second liquid from the upper part of the extraction chamber. The column normally comprises a vertical drive shaft that extends axially the length of the column. The shaft is connected to a plurality of blades which provide for the mixing of the two liquids. The blades are normally provided in the column such that there is a settling and separating space for the lighter and heavier liquids after mixing. Counter current extraction columns can be purchased in different sizes from several manufacturers, e.g. from NORMAG Labor- und Prozesstechnik GmbH, Ilmenau, Germany.

Preferably, the extraction device for use in the present invention comprises a heating device, such as a heating sheath, which can be used for controlling the temperature in the extraction device. More preferably, the heating device allows for different temperatures in different sections of the device. In other words, the heating device can be used for establishing a temperature gradient within the device. Such temperature gradients are useful for gradually heating the medium flowing through the device to the respective CPT. A temperature gradient is helpful for increasing the extent of phase separation.

The medium which flows through the extraction device is then contacted in counter current direction with a flow of a liquid surfactant which serves as an extractant. The surfactant is significantly higher concentrated than the surfactant in the medium. Preferably, the surfactants used in step (b) and (d) of the method of the invention are identical (d. The solution used in step (d) will have a significantly higher surfactant concentration compared to the medium. The concentration will be higher than 200-fold, preferably 250-fold or more, 300-fold or more, 400-fold or more, 500-fold or more, 750-fold or more, or 1000-fold or more of the surfactant's cmc. The concentration will be sufficiently high to allow phase separation upon increasing the temperature. The medium flow and the surfactant flow are adapted to allow optimal extraction efficiencies, low surfactant concentrations in the medium leaving the device and a high enrichment of hydrophobic compounds in the extract.

Preferably, the volume ratio of medium:extractant for a given retention time in the extraction device ranges from 2:1 to 100:1, and preferably is about 3:1, 4:1, 5:1, 6:1, 7:1, 8:1, 9:1, 10:1, 15:1, 20:1, 25:1, 30:1, 40:1, 50:1, 60:1, 70:1, 80:1, or 90:1.

When a vertically oriented extraction column is used, the medium containing the cells has a higher density than the surfactant solution used for extraction at cpt, it will normally be introduced in the upper section of the extraction column so that it moves downwards through the column and leaves the column via the outlet at the bottom part of the column. The surfactant solution which has the lower density is conducted into the lower part of the extraction column and moves through the column in counter current flow relative to the medium flow. It ultimately forms a separate phase in the upper part of the extraction column. Conversely, if the medium containing the cells has a lower density than the surfactant solution used for extraction, it will be introduced in the lower section of the extraction column so that it moves upwards through the column and leaves the column via the outlet at the upper part of the column. The surfactant solution which has the higher density is conducted into the upper part of the extraction column and moves downwards through the column in counter current flow relative to the medium flow.

The temperature in the extraction device is increased to a temperature which is higher than the surfactant's cloud point temperature (cpt) to induce phase separation. This will have the effect that the medium separates into two different phases: an aqueous phase containing the cells and a surfactant-rich phase containing the micelles that encompass the hydrophobic target materials. The cloud point temperature is a parameter that can normally be taken from the package insert of the respective detergent, and it can also be determined e.g. according to the Standard Test Method for Cloud Point of Nonionic Surfactants (ASTM D2024-09).

Preferably, the maximum temperature in the extraction device will be about 3°C, preferably about 4°C, about 5°C, about 6°C, about 7°C, about 8°C, about 9°C, or about 10°C higher than the cpt of the surfactant. A higher temperature in the extraction device will generally improve phase separation. However, in cases where the cells in the medium shall be re-used for further rounds of cultivation, e.g. by inoculating fresh medium, care must be taken not to set a temperature which is so high that the cells are damaged.

By increasing the temperature, the micelle-containing medium separates into an aqueous phase containing the cells and a surfactant-rich phase containing the micelles. The latter phase is extracted by the highly concentrated surfactant flow which passes the medium in counter current direction. In this way, the surfactant flow is stepwise enriched for the hydrophobic target materials.

In one embodiment, the method is performed by conducting 100 L or more, 200 L or more, or 300 L or more of cell-containing medium through the extraction device per hour, and at the same time, conducting 10 L or less, 7.5 L or less, or 5 L or less surfactant solution through the extraction device per hour.

Subsequently, the surfactant phase which has passed through the extraction device is recirculated back into the extraction device so that another volume of the culture medium can be extracted. The surfactant phase which has passed through the extraction device can be recirculated into the extraction device until a saturation of the phase with hydrophobic target compounds is achieved. By recirculation of the surfactant phase into the extraction device, the amount of surfactant required for extraction is dramatically reduced compared to batch extraction. In this way, the complete volume of the cell culture can be extracted by conducting it through the counter current extraction device before the surfactant-rich phase containing the micelles is harvested.

In one preferred embodiment, the aqueous phase containing the cells has a surfactant concentration of 1-200-fold of the surfactant's cmc after passing through the extraction device. This has the advantage that the medium and the cells can be recirculated into the culturing vessel for further culturing rounds, e.g. for the inoculation of fresh medium. The surfactant concentration of the aqueous phase that has passed through the extraction device can be adapted, e.g. by modifying the temperature in the device or the velocity of medium and surfactant flow. For example, if the velocity of the flows is selected such that the retention time is short, the aqueous phase that has passed through the extraction device will have a higher surfactant concentration. Longer retention times will decrease the surfactant concentration, since the surfactant will be transferred from the aqueous phase to the surfactant phase.

A schematic view of this embodiment is shown in Figure 2. It can be seen that the medium containing the cells producing the hydrophobic compounds is conducted from a cell culture vessel (6) via an inlet into the lower part of a counter current extraction column (5). After floating through the counter current extraction column (5), the medium leaves the column at the upper part and is conducted back into the cell culture vessel (6). In this way a cyclic medium flow (1) is established. At the same time, a cyclic surfactant (2) is established by conducting the highly concentrated surfactant solution via a pump (3) into the upper part of the counter current extraction column (5). After being contacted with the medium in the counter current extraction column (5), the surfactant which has been enriched for hydrophobic compounds leaves the column via an outlet at the lower part and is conducted back into the separation container (4). It is to be noted that in the depicted embodiment, the surfactant has a higher density than the medium and is therefore introduced in the upper part of the column. In cases where the medium has a higher density, the medium would be introduced in the upper part.

In other words, the method of the invention can be carried out such that both the cell-containing medium and the surfactant used for extraction are recirculated one or several times into the counter current extraction device, e.g. the counter current extraction column. Preferably, the cell-containing medium is circulated through the counter current extraction device at least 2 times, at least 3 times, at least 4 times, at least 5 times, at least 6 times, at least 7 times, at least 8 times, at least 9 times, or at least 10 times. Likewise, it is preferred that the surfactant used for extraction is circulated through the counter current extraction device at least 2 times, at least 3 times, at least 4 times, at least 5 times, at least 6 times, at least 7 times, at least 8 times, at least 9 times, or at least 10 times.

Once the surfactant phase has been enriched with the micelles from the cell-containing medium, the surfactant phase can be harvested and the lipophilic compounds can, if necessary, be further enriched or purified from the surfactant phase. Preferably, the lipophilic compounds are separated from the surfactant molecules. Preferably, the lipophilic compounds are enriched or purified by separating at least 90%, at least 95%, at least 98%, at least 99%, or more of the surfactant molecules from the lipophilic target compounds. Methods for purifying lipophilic compounds from surfactants are known in the art and include, e.g. chromatographic methods.

In an alternative embodiment, the surfactant phase which has been enriched with the micelles from the cell-containing medium is directly used as an additive for the preparation of food items, e.g. health food, cosmetic or pharmaceutical products. This means that the surfactant phase is not subjected to further enrichment or purification processes. In such an embodiment, the surfactant used in the process of the invention will preferably have regulatory approval for being used as additive in food items, or in cosmetic or pharmaceutical products.

### DESCRIPTION OF THE FIGURES

Figure 1 shows a counter current extraction column for use in the method of the invention.
Figure 2 shows an embodiment of the method of the invention in which both the medium flow and the surfactant flow are circulated through the extraction column.

### EXAMPLES

The following example shows the in situ micellar extraction of a microalgae culture with the surfactant Rokanol NL 5
The cultivation of the microalgae *Acutodesmus obliquus* was done in parallel batches, each batch consisting of 32 flat panels mounted at the southeast and southwest oriented facades of a residential building in Hamburg (Germany). The photobioreactors in each of the parallel batches covered a photosynthetic active area of about 50 m², holding a volume of culture medium of about 900 L. Nitrogen, phosphor and CO₂ were added to the culture media to cover the nutrient demand of the algae. Culturing was performed at a temperature between 20-25°C and a pH value of about 7 until the cell density in the media reached both 1 g dry weight/L.

To one of the parallels Rokanol NL 5 was added to a concentration of 170 cmc while the other remained unchanged as a control and culturing of both was continued for another 72 hours before continuous extraction started.

For continuous in situ extraction a counter current extraction column having a volume of 32.5 L was used.

The flow of culture medium into and out of the extraction column was kept at 7 L/h thus obtaining a retention time of 4.6 hours. The counter current flow of the surfactant in and out of the column was set to 2.33 L/h. Thus a ratio of flow of medium to the flow of surfactant of 3 was established.

The temperature in the column was set to 40°C. After 5.5 hours of continuous extraction 0.5 L of surfactant was obtained and analysed for pigments using High Pressure Liquid Chromatography (HPLC). In addition the pigment concentration in both culture media (with and without Rokanol addition) before extraction was analysed in order to determine the total amount of pigments available for extraction (Table 1) and the efficiency of extraction, i.e. the amount of pigments in the extract in relation to the pigments available in the medium (Table 2).

| Medium | Neoxanthin | Violaxanthin | Lutein | Chl. b | Chl. a |
|---|---|---|---|---|---|
| with Rokanol | 0,31 | 0,22 | 1,40 | 3,29 | 5,99 |
| Control | 0,23 | 0,17 | 0,96 | 2,44 | 5,17 |
| % enrichment | 27,6 | 26,2 | 31,4 | 25,9 | 13,7 |

Table 1. Total amounts of pigments detected in the culture medium (mg/L) prior to extraction and the percentage of enrichment in the medium with Rokanol addition compared to the control. Data show that Rokanol addition during cultivation increased the amount of pigments up to 28% in comparison to the control.

| Medium | Neoxanthin | Violaxanthin | Lutein | Chl. b | Chl. a |
|---|---|---|---|---|---|
| with Rokanol | 36,4 | 48,2 | 25,3 | 87,2 | 86,6 |
| Control | 5,6 | 18,5 | 11,6 | 23,6 | 23,2 |

Table 2. Efficiency of in situ extraction in the extraction column given as percentage of the total amounts of pigments present in the medium (Table 1). Data show that the efficiency in the medium with Rokanol was much higher than in the control owing to the fact that most of the pigments were present in the micelles and not extracted from the algae cells.

## Claims

1. Method for the micelle-mediated in situ extraction of hydrophobic compounds from cells, comprising
(a) culturing cells in an aqueous medium such that hydrophobic compounds are produced;
(b) adding to the cell-containing medium a surfactant in a concentration of 1-200-fold of the surfactant's critical micelle forming concentration (cmc) to form micelles that contain hydrophobic compounds;
(c) conducting the cell-containing medium into a counter current extraction device to provide a medium flow;
(d) contacting the medium flow in said extraction device with a flow of a liquid surfactant which comprises a concentration of more than 200-fold of the surfactant's cmc and which is conducted into said extraction device in counter current direction relative to the medium flow;
(e) increasing the temperature in the said extraction device to a temperature which is higher than the surfactant's cloud point temperature (cpt) to separate an aqueous phase containing the cells from a surfactant-rich phase containing the micelles;
(f) circulating the surfactant-rich phase which has passed through said extraction device back into the extraction device.

2. Method according to claim 1, wherein said aqueous phase containing the cells has a surfactant concentration of between 1-200-fold of the surfactant's cmc after passing through the extraction device.

3. Method according to claim 1 or 2, wherein said cells are algae, plant, bacterial or fungal cells.

4. Method according to any of claims 1-3, wherein said algae are from the genus Dunaliella, Haematococcus, Chlorella, Coccomyxa, Coelastrella or Scenedesmus.

5. Method according to claim 4, wherein said algae are from the species Dunaliella salina, Haematococcus pluvialis, Chlorella vulgaris, Coccomyxa acidophila, Coelastrella striolata or Scenedesmus almeriensis.

6. Method according to any of claims 1-5, wherein said surfactant is a nonionic surfactant, preferably a nonionic surfactant which is not toxic for the cells.

7. Method according to claim 6, wherein said method further comprises step (e) where the surfactant-rich phase which has passed several times through said extraction device is obtained and, preferably, processed without separating the surfactant from the lipophilic compounds.

8. Method according to any of claims 1-4, wherein said hydrophobic compounds comprise fatty acids, hydrocarbons, waxes, sterols, ketones, pigments, proteins, or enzymes.

9. Method according to claim 8, wherein said hydrophobic compounds comprise compounds of nutritional value, compounds having pro- and/or prebiotic activity, such as unsaturated fatty acids, pharmaceutically active compounds, antibiotic compounds, antiviral compounds, anti-inflammatory compounds, and antioxidants.

10. Method according to any of claims 1-8, wherein the surfactant-rich phase containing the micelles is obtained after the complete volume of the cell culture has been conducted through the counter current extraction device.

11. Method according to any of claims 1-10, wherein the counter current extraction device has a volume of between 2-50 L, preferably 35 L.

12. Method according to any of claims 1-11, wherein the counter current extraction device is a column has a diameter of between about 0.1-1.0 m, preferably about 0.5 m.

13. Method according to any of claims 1-12, wherein said bacteria are Cyanobacteria or Rhodospirillales.

14. Method according to any of claims 1-13, wherein 300 L cell-containing medium or more are conducted through the extraction device per hour.

15. Method according to any of claims 1-14, wherein 5-10 L surfactant solution are conducted through the extraction device per hour.

## Patentansprüche

1. Verfahren zur Mizellen-vermittelten in-situ-Extraktion von hydrophoben Verbindungen aus Zellen, bei dem man
(a) Zellen in einem wässrigen Medium so kultiviert, dass hydrophobe Verbindungen entstehen;
(b) dem zellhaltigen Medium ein Tensid in einer Konzentration zugibt, die dem 1-200-fachen der kritischen Mizellenbildungskonzentration (critical micelle forming concentration; CMC) des Tensids entspricht, um Mizellen zu bilden, welche hydrophobe Verbindungen enthalten;
(c) das zellhaltige Medium in eine Gegenstrom-Extraktionsvorrichtung leitet, um einen Medium-Strom zu erzeugen;
(d) den Medium-Strom in der Extraktionsvorrichtung mit einem Strom eines flüssigen Tensids in Kontakt bringt, der eine Konzentration aufweist, die dem mehr als 200-fachen der CMC des Tensids entspricht, und der relativ zum Medium-Strom in Gegenstromrichtung in die Extraktionsvorrichtung geleitet wird;
(e) die Temperatur in der genannten Extraktionsvorrichtung auf eine Temperatur erhöht, die höher ist als die Trübungspunkttemperatur (cloud point temperature; CPT) des Tensids, um eine wässrige Phase, welche die Zellen enthält, von einer Tensidreichen Phase, welche die Mizellen enthält, zu trennen;
(f) die Tensid-reiche Phase, welche die Extraktionsvorrichtung durchlaufen hat, zurück in die Extraktionsvorrichtung zirkuliert.

2. Verfahren nach Anspruch 1, bei dem die wässrige Phase, welche die Zellen enthält, nach Durchlaufen der Extraktionsvorrichtung eine Tensid-Konzentration aufweist, die dem 1-200-fachen der CMC des Tensids entspricht.

3. Verfahren nach Anspruch 1 oder 2, bei dem die Zellen Algen-, Pflanzen-, Bakterien- oder Pilzzellen sind.

4. Verfahren nach einem der Ansprüche 1-3, bei dem die Algen zur Gattung Dunaliella, Haematococcus, Chlorella, Coccomyxa, Coelastrella oder Scenedesmus gehören.

5. Verfahren nach Anspruch 4, wobei die Algen zur Spezies Dunaliella salina, Haematococcus pluvialis, Chlorella vulgaris, Coccomyxa acidophila, Coelastrella striolata oder Scenedesmus almeriensis gehören.

6. Verfahren nach einem der Ansprüche 1-5, bei dem das Tensid ein nicht-ionisches Tensid ist, vorzugsweise ein nicht-ionisches Tensid, das für die Zellen nicht toxisch ist.

7. Verfahren nach Anspruch 6, bei dem das Verfahren ferner Schritt (e) umfasst, bei dem man die Tensid-reiche Phase, welche die Extraktionsvorrichtung mehrfach durchlaufen hat, gewinnt und vorzugsweise verarbeitet, ohne das Tensid von den lipophilen Verbindungen zu trennen.

8. Verfahren nach einem der Ansprüche 1-4, bei dem die hydrophoben Verbindungen Fettsäuren, Kohlenwasserstoffe, Wachse, Sterole, Ketone, Pigmente, Proteine oder Enzyme umfassen.

9. Verfahren nach Anspruch 8, bei dem die hydrophoben Verbindungen Verbindungen mit Nährwert, Verbindungen mit pro- und/oder präbiotischer Aktivität, wie beispielsweise ungesättigte Fettsäuren, pharmazeutisch aktive Verbindungen, antibiotische Verbindungen, antivirale Verbindungen, entzündungshemmende Verbindungen und Antioxidantien umfassen.

10. Verfahren nach einem der Ansprüche 1-8, bei dem die Tensid-reiche Phase, welche die Mizellen enthält, gewonnen wird, nachdem das gesamte Volumen der Zellkultur durch die Gegenstrom-Extraktionsvorrichtung geleitet worden ist.

11. Verfahren nach einem der Ansprüche 1-10, bei dem die Gegenstrom-Extraktionsvorrichtung ein Volumen von 2-50 L aufweist, vorzugsweise 35 L.

12. Verfahren nach einem der Ansprüche 1-11, bei dem die Gegenstrom-Extraktionsvorrichtung eine Säule mit einem Durchmesser von etwa 0,1 bis 1,0 m ist, vorzugsweise von etwa 0,5 m.

13. Verfahren nach einem der Ansprüche 1-12, bei dem die Bakterien Cyanobakterien oder Rhodospirillales sind.

14. Verfahren nach einem der Ansprüche 1-13, bei dem 300 L zellhaltiges Medium oder mehr pro Stunde durch die Extraktionsvorrichtung geleitet werden.

15. Verfahren nach einem der Ansprüche 1-14, bei dem 5-10 L Tensid-Lösung pro Stunde durch die Extraktionsvorrichtung geleitet werden.

## Revendications

1. Procédé visant à extraire in situ de cellules, au moyen de micelles, des composés hydrophobes, comportant les étapes suivantes :
a) cultiver des cellules dans un milieu aqueux, de manière à ce que soient produits des composés hydrophobes,
b) ajouter, au milieu contenant les cellules, un tensioactif en une concentration valant de 1 à 200 fois la concentration critique (Ccfm) de formation de micelles de ce tensioactif, pour que se forment des micelles contenant les composés hydrophobes,
c) envoyer le milieu contenant les cellules dans un dispositif d'extraction à contre-courant, en tant que courant de milieu,
d) mettre ce courant de milieu en contact, dans ledit dispositif d'extraction, avec un courant de liquide tensioactif, qui contient du tensioactif en une concentration valant plus de 200 fois la Ccfm de ce tensioactif et qui est envoyé dans ledit dispositif d'extraction à contre-courant par rapport au courant de milieu,
e) élever la température régnant dans ledit dispositif d'extraction jusqu'à une température supérieure à la température (Tpt) du point de trouble du tensioactif, afin de séparer une phase aqueuse contenant les cellules d'avec une phase riche en tensioactif et contenant les micelles,
f) et recycler cette phase riche en tensioactif, après son passage dans ledit dispositif d'extraction, en la renvoyant dans celui-ci.

2. Procédé conforme à la revendication 1, dans lequel ladite phase aqueuse contenant les cellules, après être passée dans ledit dispositif d'extraction, contient du tensioactif en une concentration valant de 1 à 200 fois la Ccfm de celui-ci.

3. Procédé conforme à la revendication 1 ou 2, dans lequel lesdites cellules sont des cellules d'algues, ou des cellules végétales, bactériennes ou fongiques.

4. Procédé conforme à l'une des revendications 1 à 3, dans lequel lesdites algues sont du genre *Dunaliella, Haematococcus, Chlorella, Coccomyxa, Coelastrella* ou *Scenedesmus.*

5. Procédé conforme à la revendication 4, dans lequel lesdites algues sont de l'espèce *Dunaliella salina, Haematococcus pluvialis, Chlorella vulgaris, Coccomyxa acidophila, Coelastrella striolata* ou *Scenedesmus almeriensis.*

6. Procédé conforme à l'une des revendications 1 à 5, dans lequel ledit tensioactif est un tensioactif non-ionique, et de préférence, un tensioactif non-ionique qui n'est pas toxique pour les cellules.

7. Procédé conforme à la revendication 6, lequel procédé comporte en outre une étape (e) dans laquelle on récupère la phase riche en tensioactif qui est passée plusieurs fois dans ledit dispositif d'extraction et on la traite, de préférence, sans séparer le tensioactif d'avec les composés lipophiles.

8. Procédé conforme à l'une des revendications 1 à 4, dans lequel lesdits composés hydrophobes comprennent des acides gras, des hydrocarbures, des cires, des stérols, des cétones, des pigments, des protéines ou des enzymes.

9. Procédé conforme à la revendication 8, dans lequel lesdits composés hydrophobes comprennent des composés à valeur nutritionnelle, des composés ayant une activité de probiotique et/ou de prébiotique, tels des acides gras insaturés, des composés pharmacologiquement actifs, des composés antibiotiques, des composés antiviraux, des composés anti-inflammatoires, et des antioxydants.

10. Procédé conforme à l'une des revendications 1 à 8, dans lequel c'est après avoir fait passer tout le volume de la culture de cellules dans le dispositif d'extraction à contre-courant qu'on récupère la phase riche en tensioactif et contenant les micelles.

11. Procédé conforme à l'une des revendications 1 à 10, dans lequel le volume du dispositif d'extraction à contre-courant vaut de 2 à 50 L, et de préférence 35 L.

12. Procédé conforme à l'une des revendications 1 à 11, dans lequel le dispositif d'extraction à contre-courant est une colonne dont le diamètre vaut à peu près de 0,1 à 1,0 m, et de préférence, environ 0,5 m.

13. Procédé conforme à l'une des revendications 1 à 12, dans lequel lesdites bactéries sont des cyanobactéries ou des rhodospirillales.

14. Procédé conforme à l'une des revendications 1 à 13, dans lequel on fait passer dans le dispositif d'extraction, par heure, 300 L ou plus de milieu contenant des cellules.

15. Procédé conforme à l'une des revendications 1 à 14, dans lequel on fait passer dans le dispositif d'extraction, par heure, de 5 à 10 L de solution de tensioactif.
